Europäisches Patentamt

European Patent Office

Office européen des brevets

(19)

(11) Veröffentlichungsnummer: **0 163 250 B1**

## EUROPÄISCHE PATENTSCHRIFT

(12)

(45) Veröffentlichungstag der Patentschrift: **13.05.92**

(51) Int. Cl.⁵: **C07D 221/14**

(21) Anmeldenummer: **85106280.2**

(22) Anmeldetag: **22.05.85**

(54) **Verfahren zur Herstellung der trockenen Alkalimetallsalze des 1,8-Naphthalsäureimids.**

(30) Priorität: **30.05.84 DE 3420161**

(43) Veröffentlichungstag der Anmeldung:
**04.12.85 Patentblatt 85/49**

(45) Bekanntmachung des Hinweises auf die
Patenterteilung:
**13.05.92 Patentblatt 92/20**

(84) Benannte Vertragsstaaten:
**BE CH DE FR GB IT LI**

(56) Entgegenhaltungen:
**EP-A- 0 123 256**
**DE-B- 2 137 242**
**US-A- 3 997 572**
**US-A- 4 051 246**

**CHEMICAL ABSTRACTS, Band 54, Nr. 13, 10.
Juli 1960, Columbus, Ohio, USA, Spalte 13
073g,h; A.P. KARISHIN et. al.: "N-alkyl derivatives of naphthalimide and halonaphtalimides"**

(73) Patentinhaber: **HOECHST AKTIENGESELL-
SCHAFT
Postfach 80 03 20
W-6230 Frankfurt am Main 80(DE)**

(72) Erfinder: **Spietschka, Ernst, Dr.
Kirchweg 3
W-6270 Idstein/Taunus(DE)**
Erfinder: **Urban, Manfred
Steigerwaldstrasse 2a
W-6200 Wiesbaden(DE)**

## Beschreibung

Die vorliegende Erfindung betrifft ein verbessertes, technisch einfaches Verfahren zur Herstellung der trockenen Alkalimetallsalze des 1,8-Naphthalsäureimids in hoher Raumzeitausbeute.

Die trockenen Alkalimetallsalze des 1,8-Naphthalsäureimids sind technisch wertvolle Zwischenprodukte zur Herstellung von Perylen-3,4,9,10-tetracarbonsäurediimid (Patentanmeldungen DE-OS 3 314 430 und EP-A-0 123 256). Die Alkalimetallsalze des 1,8-Naphthalsäureimids sind bekannte Verbindungen, zu deren Herstellung folgende Verfahren in der Literatur beschrieben sind:

Gemäß dem in der US-PS 4 051 246 beschriebenen Verfahren wird das Natriumsalz des 1,8-Naphthalsäureimids in organischem Lösungsmittel durch Umsetzung von 1,8-Naphthalsäureimid mit einer methanolischen Natriumhydroxydlösung erhalten, aus der es durch Absaugen isoliert und dann getrocknet wird. Die Isolierung aus dem Lösungsmittel, sowie die Regenerierung des Lösungsmittels sind technisch aufwendige Verfahrensschritte. In der Zeitschrift Z. obs. Chim. 29 (1959) Nr. 95, Seiten 3048-3050, wird beschrieben, daß man die Alkalimetallsalze des 1,8-Naphthalsäureimids durch Umsetzung von 1,8-Naphthalsäureimid mit Alkalien in wäßriger Lösung erhalten kann. Bei diesem Verfahren müssen die Endprodukte durch Filtration aus der wäßrigen Suspension isoliert und dann getrocknet werden und das anfallende Abwasser muß aufbereitet werden. In der US-PS 3 997 572 wird die Herstellung des Kaliumsalzes des 1,8-Naphthalsäureimids durch Umsetzung von 1,8-Naphthalsäureimid mit Kaliumhydroxid in methanolischer Lösung beschrieben, wobei anschließend das Methanol durch Vakuumdestillation entfernt wird. Bei diesem Verfahren wird in großer Verdünnung im organischen Lösungsmittel gearbeitet, das durch aufwendige Destillation entfernt werden muß.

Es wurde nun gefunden, daß auf technisch äußerst einfache Weise die trockenen Alkalimetallsalze des 1,8-Naphthalsäureimids hergestellt werden können, indem man konzentrierte Alkalilaugen, wie beispielsweise konzentrierte Natronlauge oder Kalilauge, in festes 1,8-Naphthalsäureimid im molaren Verhältnis, d.h. im Molverhältnis 1:1, unter starker Rührung im Vakuum, zweckmäßigerweise bei etwa 2,66644-26,6644 $10^3$ Pa (20-200 Torr), und bei erhöhter Innentemperatur, zweckmäßigerweise bei Temperaturen von etwa 60-100°C (Trockenpfannenbedingungen) entsprechend dem Umsetzungsgrad, d.h. in Anteilen, beispielsweise durch Zutropfen und unter Vermeidung eines größeren lokalen Überschusses an Alkalilauge, an der Einlaufstelle zulaufen läßt, wobei vollständige Umsetzung, d.h. vollständige Bildung der Alkalimetallsalze des 1,8-Naphthalsäureimids erreicht wird, und anschließend ohne Zwischenisolierung der gebildeten Alkalimetallsalze trocknet.

Das aus der eingesetzten konzentrierten Alkalilauge stammende Wasser und das durch die Umsetzung des 1,8-Naphthalsäureimids mit der Alkalilauge entstehende Reaktionswasser (Neutralisationswasser) kann während oder nach vollständigem Zulauf der Alkalilauge durch Überdestillieren entfernt werden. Anstelle von trockenem 1,8-Naphthalsäureimid kann auch schwach wasserhaltiges 1,8-Naphthalsäureimid, wie es nach dem in der DE-PS 2 137 242 beschriebenen Verfahren hergstellt wird, eingesetzt werden.

Es ist als überraschend zu erachten, daß die Umsetzung des 1,8-Naphthalsäureimids in fester Form mit konzentrierten Alkalilaugen, d.h. in Gegenwart nur sehr geringer Mengen Wasser zur vollständigen Umsetzung führt, da bei den bekannten Verfahren stets in Lösung mit verdünnten Alkalien gearbeitet werden muß. Im Hinblick darauf, daß Carbonsäureimide bzw. Carbonsäureamide unter der Einwirkung von starken Alkalien bei erhöhter Temperatur verseift werden, konnte ein Gelingen der angestrebten Umsetzung in dem tatsächlichen Umfang (Erzielung quantitativer Ausbeute ohne Verseifung) nicht erwartet werden (FIESER Lehrbuch der organischen Chemie 1954, Seite 251; BEYER, Lehrbuch der organischen Chemie 1968, Seite 208).

Beispiel 1

In einen 1 l V4A-Autoklav werden 197 g 1,8-Naphthalsäureimid vorgelegt und auf 95°C erhitzt, wobei ein Vakuum von 13,3322 $10^3$ Pa (100 Torr) angelegt wird. Anschließend läßt man bei den genannten Temperatur- und Druckverhältnissen innerhalb von 5 Stunden 111 g 48%ige Kalilauge unter Rühren zutropfen, worauf man noch 6 Stunden nachrührt (bis kein Wasser mehr übergeht). Darauf entleert man den Autoklav.

Gewonnen werden 232 g 1,8-Naphthalsäureimid-Kalium, was einer Ausbeute von 98,7 % der Theorie entspricht.

Beispiel 2

In einen 1 l V4A-Autoklav werden 197 g 1,8-Naphthalsäureimid vorgelegt und auf 95°C erhitzt, wobei ein Vakuum von 13,3322 $10^3$ Pa (100 Torr) angelegt wird. Anschließend läßt man bei diesen Temperatur- und Druckverhältnissen innerhalb von 5 Stunden 121 g 33%ige Natronlauge unter Rühren zutropfen, worauf man noch 6 Stunden nachrührt (bis kein Wasser mehr übergeht). Darauf entleert man den Autoklav.

Gewonnen werden 219 g 1,8-Naphthalsäureimid-Natrium, was einer Ausbeute von 100 % der Theorie entspricht.

**Patentansprüche**

1. Verfahren zur Herstellung der trockenen Alkalimetallsalze des 1,8-Naphthalsäureimids, dadurch gekennzeichnet, daß man konzentrierte Alkalilaugen in festes 1,8-Naphthalsäureimid im molaren Verhältnis entsprechend dem Umsetzungsgrad unter starker Rührung bei einem Druck von 2,66644-26,6644 $10^3$ Pa (20-200 Torr) bei Temperaturen von 60-100°C zulaufen läßt und anschließend ohne Zwischenisolierung des gebildeten Alkalimetallsalzes trocknet.

2. Verfahren nach Anspruch 1, dadurch gekennzeichnet, daß man konzentrierte Natronlauge oder Kalilauge zulaufen läßt.

3. Verfahren nach mindestens einem der Ansprüche 1 und 2, dadurch gekennzeichnet, daß trockenes 1,8-Naphthalsäureimid eingesetzt wird.

4. Verfahren nach mindestens einem der Ansprüche 1 bis 3, dadurch gekennzeichnet, daß das eingesetzte feste 1,8-Naphthalsäureimid schwach wasserhaltig ist.

5. Verfahren nach mindestens einem der Ansprüche 1 bis 4, dadurch gekennzeichnet, daß man aus der eingesetzten konzentrierten Alkalilauge stammendes Wasser und das gebildete Reaktionswasser während oder nach der Umsetzung destillativ entfernt.

**Claims**

1. A process for the preparation of a dry alkali metal salt of 1,8-naphthalimide, which comprises allowing a concentrated alkali metal hydroxide solution to run into solid 1,8-naphthalimide in a molar ratio at a rate corresponding to the degree of reaction, with vigorous stirring at a pressure of 2.66644 - 26.6644 $10^3$ Pa (20-200mm Hg) and at temperatures of 60 - 100°C, and then carrying out drying without intermediate isolation of the alkali metal salt formed.

2. The process as claimed in claim 1, wherein concentrated sodium hydroxide solution or potassium hydroxide solution is allowed to run in.

3. The process as claimed in at least one of claims 1 and 2, wherein dry 1,8-naphthalimide is employed.

4. The process as claimed in at least one of claims 1 to 3, wherein the solid 1,8-naphthalimide employed contains a small amount of water.

5. The process as claimed in at least one of claims 1 to 4, wherein water originating from the concentrated alkali metal hydroxide solution employed and the water of reaction formed are removed by distillation during or after the reaction.

**Revendications**

1. Procédé pour préparer les sels de métaux alcalins secs du naphtalène-1,8-dicarboximide, procédé caractérisé en qu'on introduit des solutions concentrées d'alcalis dans du naphtalène-1,8-dicarboximide solide, dans le rapport molaire, à un débit adapté au taux de réaction, tout en agitant énergiquement, sous une pression de 2,66644 à 26,6644 $10^3$ Pa (soit de 20 à 200 torr), à des températures de 60 à 100°C, puis, sans l'isoler intermédiairement, on sèche le sel de métal alcalin formé.

2. Procédé selon la revendication 1 caractérisé en ce qu'on introduit une solution concentrée d'hydroxyde de sodium ou d'hydroxyde de potassium.

3. Procédé selon au moins une des revendications 1 et 2, caractérisé en ce qu'on utilise le naphtalène-1,8-dicarboximide à l'état sec.

4. Procédé selon au moins une des revendications 1 à 3, caractérisé en ce que le naphtalène-1,8-dicarboximide solide mis en jeu renferme une petite quantité d'eau.

5. Procédé selon au moins une des revendications 1 à 4, caractérisé en ce qu'on élimine par distillation, pendant ou après la réaction, l'eau provenant de la solution concentrée d'alcali mise en jeu et l'eau engendrée par la réaction.